# EUROPÄISCHE PATENTSCHRIFT

(11) **EP 2 628 503 B1**
(45) Veröffentlichungstag und Bekanntmachung des Hinweises auf die Patenterteilung: **24.02.2016**
(21) Anmeldenummer: 12196255.9
(22) Anmeldetag: 10.12.2012
(51) Int. Cl.: A61N 1/365, A61N 1/368

(54) **Herzstimulator für eine kardiale Kontraktilitätsmodulation**
Cardiac stimulator for cardiac contractility modulation
Stimulateur cardiaque pour une modulation de la contractilité cardiaque

(30) Priorität: 16.02.2012 US 201261599432 P
(43) Veröffentlichungstag der Anmeldung: 21.08.2013
(73) Patentinhaber: BIOTRONIK SE & Co. KG, 12359 Berlin (DE)
(72) Erfinder: Dörr, Thomas, 12437 Berlin (DE)
(74) Vertreter: Galander, Marcus

(56) Entgegenhaltungen:
- US-A1- 2003 120 318
- US-A1- 2006 247 699
- US-A1- 2009 082 825
- US-A1- 2010 069 977
- US-B1- 6 360 126
- US-B1- 6 370 430

## Beschreibung

Die Erfindung betrifft einen Herzstimulator mit wenigstens einer Stimulationseinheit, die über wenigstens eine Elektrodenleitung mit wenigstens drei Stimulationselektrodenpolen verbunden oder zu verbinden ist und die ausgebildet ist, über wenigstens zwei Stimulationselektrodenpole unterschwellige Stimulationsimpulse für eine kardiale Kontraktionsmodulationstherapie (CCM) abzugeben.

Implantierbare Herzstimulatoren in Form von Herzschrittmachern oder Cardiovertern/Defibrillatoren sind grundsätzlich bekannt. Solche Herzstimulatoren sind in der Regel an Elektrodenleitungen angeschlossen, die in einer Kammer eines Herzens oder in unmittelbarer Nähe Stimulations- und optional zusätzliche Defibrillationselektroden besitzen. Über eine Stimulationselektrode kann ein Herzschrittmacher einen elektrischen Stimulationsimpuls an das Muskelgewebe einer Herzkammer abgeben, um so eine stimulierte Kontraktion der Herzkammer hervorzurufen, sofern der Stimulationsimpuls eine ausreichende Intensität besitzt und das Herzmuskelgewebe (Myokard) sich nicht gerade in einer refraktären Phase befindet. Eine derartig stimulierte Kontraktion einer Herzkammer wird im Rahmen dieser Beschreibung als stimuliertes Ereignis bezeichnet, ein Stimulationsimpuls, der eine ausreichende Intensität besitzt, eine stimulierte Kontraktion einer Herzkammer hervorzurufen, wird als "überschwellig" bezeichnet. Kommt es zu einer natürlichen Kontraktion der Herzkammer, wird dies im Rahmen dieser Beschreibung als Eigenaktion oder als natürliches oder intrinsisches Ereignis bezeichnet. Eine Kontraktion beispielsweise des rechten Atriums eines Herzens wird als atriales Ereignis bezeichnet, welches beispielsweise ein natürliches atriales Ereignis sein kann, oder - im Falle eines atrialen Herzschrittmachers - auch ein stimuliertes atriales Ereignis. Im gleichen Sinne können natürliche (intrinsische) und stimulierte linksventrikuläre und rechtsventrikuläre Ereignisse unterschieden werden.

Eine lokale Erregung des Myokards breitet sich vom Erregungsort ausgehend per Reizleitung im Myokard aus und führt zu einer Depolarisation der Muskelzellen und damit zu einer Kontraktion des Myokards. Nach kurzer Zeit kommt es zu einer Repolarisation der Muskelzellen und damit zu einer Entspannung des Myokards. Während der Phase der Depolarisation sind die Herzmuskelzellen für eine Erregung unempfindlich, d.h. refraktär. Diese Zeit wird als Refraktärzeit bezeichnet. Die mit der Depolarisation und Repolarisation einhergehenden elektrischen Potentiale können wahrgenommen und deren zeitlicher Verlauf - als Elektrokardiogramm bezeichnet - ausgewertet werden.

In einem Elektrokardiogramm sind mit einer Kontraktion des Ventrikels einhergehende, eine Depolarisation der Herzmuskelzellen widerspiegelnde Aktionspotentiale als sogenannte Q-Zacke zu erkennen, während sich die mit der Entspannung des Myokards einhergehende Repolarisierung der Herzmuskelzellen in einer sogenannten T-Welle widerspiegelt.

Beim gesunden Menschen wird der jeweilige Herzrhythmus durch den vom autonomen Nervensystem gesteuerten Sinusknoten bestimmt. Dieser erregt per Reizleitung das rechte Atrium eines menschlichen Herzens und weiter über den AV-Knoten den (rechten) Ventrikel des Herzens. Ein vom Sinusknoten ausgehender natürlicher Herzrhythmus wird daher auch als Sinusrhythmus bezeichnet und führt zu jeweils natürlichen Kontraktionen der jeweiligen Herzkammer, die als natürliche (intrinsische) Ereignisse erfasst werden können.

Das Erfassen solcher natürlicher (intrinsischer) Ereignisse erfolgt durch Ableiten der elektrischen Potenziale des Myokards der jeweiligen Herzkammer mit Hilfe von Wahrnehmungselektroden, die Teil einer entsprechenden Elektrodenleitung sind. Dabei können die Wahrnehmungselektroden gleichzeitig die Stimulationselektroden sein und abwechselnd als Stimulations- und als Wahrnehmungselektroden verwendet werden. Typischerweise ist für das Sensing - d.h. die Wahrnehmung intrinsischer Ereignisse - ein Wahrnehmungselektrodenpaar vorgesehen, das von zwei benachbarten Elektroden, nämlich einer Spitzenelektrode (Tip-Elektrode) und einer Ringelektrode, gebildet ist, von denen die Spitzenelektrode auch als Stimulationselektrode dient. Auf diese Weise erfolgt eine bipolare Ableitung eines intrakardialen Elektrokardiogramms (IEGM). Dabei erfolgt das Sensing und die Stimulation im Ventrikel mit Hilfe einer ventrikulären Elektrodenleitung und die Stimulation und das Sensing im Atrium (im rechten Atrium) mit einer atrialen Elektrodenleitung, die separat an den jeweiligen Herzstimulator angeschlossen sind. Zusätzlich kann auch eine linksventrikuläre Elektrodenleitung vorgesehen sein, die typischer Weise über den Coronarsinus und eine von diesem abzweigende Lateralvene in die Nähe des linken Ventrikels ragt und dort eine kleinflächige Stimulations- und/oder Wahrnehmungselektrode aufweisen kann.

In Bezug auf die hierin verwendeten Bezeichnungen sei darauf hingewiesen, dass im Rahmen dieses Textes mit den Begriffen Stimulations- oder Wahrnehmungselektrode ein jeweiliger Elektrodenpol an einer Elektrodenleitung gemeint ist, also derjenige Teil einer Elektrodenleitung, über den Stimulationsimpulse abgegeben oder elektrische Potentiale aufgenommen werden. Es sei auch darauf hingewiesen, dass es auch üblich ist, mit "Stimulationselektrode" eine der Stimulation dienende Elektrodenleitung zu bezeichnen.

Die Wahrnehmungselektroden sind im Betrieb des Herzstimulators mit entsprechenden Wahrnehmungseinheiten verbunden, die ausgebildet sind, ein jeweiliges über eine Wahrnehmungselektrode (bzw. ein Wahrnehmungselektrodenpaar) aufgenommenes Elektrokardiogramm auszuwerten und insbesondere intrinsische atriale bzw. ventrikuläre Ereignisse zu detektieren, d.h. natürliche atriale oder ventrikuläre Kontraktionen. Dies geschieht beispielsweise durch Schwellwertvergleich, d.h. ein intrinsisches Ereignis wird detektiert, wenn ein jeweiliges intrakardiales Elektrokardiogramm einen geeignet vorgegebenen Schwellwert überschreitet.

Aus der Frequenz, mit der atriale bzw. ventrikuläre Ereignisse aufeinander folgen, kann die jeweilige intrinsische atriale Herzrate (atriale Frequenz) bzw. ventrikuläre Herzrate (Ventrikelfrequenz) abgeleitet und somit beispielsweise Tachykardien detektiert werden.

Das Erfassen natürlicher Ereignisse dient bei bekannten Demand-Schrittmachern außerdem der Unterdrückung (Inhibierung) der Abgabe von Stimulationsimpulsen an eine entsprechende Herzkammer, falls das natürliche Ereignis in einem Zeitfenster vor der geplanten Abgabe eines Stimulationsimpulses an diese Herzkammer erfasst wird. Bei ratenadaptiven Herzschrittmachern wird der Zeitpunkt der Abgabe eines jeweiligen Stimulationsimpulses in Abhängigkeit einer jeweiligen Stimulationsrate geplant, die dem physiologischen Bedarf eines Patenten entsprechen soll, also bei größerer Anstrengung beispielsweise höher ist. Hierzu kann ein Herzstimulator mit einem oder mehreren Aktivitätssensoren ausgestattet sein, der beispielsweise ein nachfolgend näher beschriebener CLS-Sensor sein kann.

Die natürliche Einwirkung des autonomen Nervensystems auf die Herzrate (Herzfrequenz), die durch einen ratenadaptiven Herzstimulator nachgebildet wird, wird als Chronotropie bezeichnet.

Neben der Chronotropie wird die Herzleistung auch von dessen Kontraktilität bestimmt, deren Beeinflussung als Inotropie bezeichnet wird.

Zur Bestimmung der Kontraktilität eines Herzen ist es bekannt, in einem Gehäuse eines Herzstimulators (z.B. eines implantierbaren Herzschrittmachers) eine Impedanz- oder Leitfähigkeitsmesseinheit anzuordnen, die ausgebildet ist, ein uni- oder bipolares Impedanz-oder Leitfähigkeitsverlaufssignal zu erzeugen. Dazu werden während wenigstens eines Herzzyklus mehrere Impedanz- oder Leitfähigkeitswerte oder ein entsprechender Impedanz- oder Leitfähigkeitsverlauf gemessen. Dies geschieht entweder unipolar durch Messen zwischen einer Neutralelektrode und einer Messelektrode oder zwischen zwei Messelektroden. Außerdem ist in dem Gehäuse eine Auswerteeinheit angeordnet, die zum Auswerten des Impedanz- oder Leitfähigkeitsverlaufs und zum Ableiten eines Kontraktilitätswertes aus dem Impedanz- oder Leitfähigkeitsverlauf dient. Elektrotherapiegeräte, die die Kontraktilität eines Herzens ermitteln können, bieten die Möglichkeit, eine von dem Elektrotherapiegerät abzugebende Therapie an den jeweiligen Kontraktilitätszustand des Herzens des Patienten anzupassen.

Wie angedeutet beschreibt die Kontraktilität einen inotropen Zustand eines Herzens. Die Kontraktilität beeinflusst die Kraft und die Geschwindigkeit einer myokardialen Kontraktion. Die Kontraktilität wird durch drei Mechanismen gesteuert:
Eine direkte Steuerung durch das autonome Nervensystem (ANS),
den sogenannten Starling-Mechanismus und
den sogenannten Bowditch-Effekt (Kraft-Frequenzkopplung).

Der Hauptmechanismus, die Steuerung der Kreislaufregulation durch das autonome Nervensystem, vergrößert die Kontraktilität und die Herzrate, wenn ein erhöhter metabolischer Bedarf vorliegt, beispielsweise bei körperlicher oder physischer Anstrengung, um eine geeignete Blutversorgung sicherzustellen. Beim gesunden Menschen bewirkt die Inotropie des Herzens somit einen Anstieg der Kontraktilität infolge erhöhten physiologischen Bedarfs.

Bei Patienten mit chronischem Herzversagen (Chronic Heart Failure, HF) nimmt die myokardiale Kontraktilität bis auf ein niedriges Niveau ab und die interventrikuläre Synchronisation wird verschlechtert. Dies wird von einem geringen Auswurfanteil (Ejection Fraction, EF) begleitet sowie von einer geringen Lebensqualität und einer hohen Sterblichkeit. HF kommt in der Bevölkerung häufig vor. In jüngerer Zeit werden HF-Patienten mit Resynchronisationstherapie-Geräten, beispielsweise 3-Kammer-Herzschrittmachern oder Defibrillatoren, behandelt. Ziel einer solchen Schrittmachertherapie ist es, die zwei Ventrikel eines Herzens durch biventrikuläre Stimulation zu synchronisieren, um das Zeitverhalten der Herzkammern und damit die Herzleistung zu verbessern. Eine derartige Therapie wird auch als kardiale Resynchronisationstherapie (Cardiac Resynchronisation Therapy, CRT) bezeichnet. Die kardiale Resynchronisationstherapie (CRT) ist hinreichend bekannt und wird z.B. von den BIOTRONIK CRT-D Implantaten (Lumax HF-T) angeboten.

Die kardiale Resynchronisationstherapie (Cardiac Resynchronisation Therapy, CRT) ist eine spezielle Form des allgemeineren kardialen Rhythmus-Managements (CCM), welches beispielsweise auch eine einfache Stimulation nur eines Ventrikels zur Behandlung beispielsweise einer Bradykardie einschließt. Ein CRM-Stimulator kann somit auch ein Einkammer-Herzschrittmacher sein.

Da die Kontraktilität des Herzens physiologisch vom autonomen Nervensystem gesteuert wird, kann die Erfassung der Kontraktilität auch zum Einstellen einer physiologisch adäquaten Stimulationsrate bei ratenadaptiven Herzschrittmachern genutzt werden. Diese weiter vorne bereits angesprochene Art der Stimulationsratensteuerung ist auch als Closed Loop Stimulation (CLS) bekannt.

Für die CLS wird der intrakardiale Impedanzverlauf nach Beginn der Ventrikelkontraktion gemessen. Diese Messung erfolgt sowohl bei intrinsischen als auch bei stimulierten Ereignissen. Es besteht eine direkte Abhängigkeit zwischen dem rechtsventrikulären Impedanzverlauf und der Kontraktionsdynamik. Die Kontraktionsdynamik wiederum ist ein Parameter für die Stimulation des Herzens durch den Sympathikus.

Die Closed Loop Stimulation dient wie gesagt der Stimulationsratensteuerung bei einem ratenadaptiven Herzschrittmacher.

Zur Steigerung der Kontraktilität einer Herzkammer ist die sogenannte kardiale Kontraktilitätsmodulationstherapie (Cardiac Contractility Modulation: CCM) bekannt.

Hierfür bietet beispielsweise die Firma Impulse Dynamics ein sogenanntes OPTIMIZER-System zur CCM-Therapie an. Dieses System umfasst einen Stimulationsimpulsgenerator, verbunden mit 3 Elektroden, von denen im Betrieb eine im Atrium und am Septum des rechten Ventrikels eines Patienten angeordnet sind. Das Therapieprinzip beruht auf einer Abgabe von biphasischen Stimulationspulsen mit einer Amplitude von 7V bis 10V und einer Gesamtimpulsdauer von ∼20ms in die absolute Refraktärzeit des Ventrikels mit dem Ziel, die Kontraktilität zu steigern. Die Therapie wird dabei für bestimmte Zeiteinheiten des Tages (z.B. abwechselnd jeweils 1h ein, 1h aus) abgegeben.

Das Prinzip der kardialen Kontraktilitätsmodulationstherapie ist unter anderem auch in US 6,317,631 B1 beschrieben.

Die Wirkung der CCM-Therapie beruht - so wird derzeit vermutet - in einer Modifikation des zellulären Kalziumionenaustauschs und führt so zu einer gesteigerten Kontraktionskraft, die auch bei einem vorliegenden Vorhofflimmern einen therapeutischen Benefit zur Folge haben sollte. Dies ist zwar klinisch noch nicht nachgewiesen, ist pathophysiologisch aber nachvollziehbar.

US 2010/0069977 A1, US 2010/0069980 A1, US 2010/0069984 A1, US 2010/0069985 A1 beschreiben Methoden, um eine CCM-Stimulation bedarfsgerecht abzugeben. Hier wird der Einsatz von physiologischen Sensoren, Nieren- oder Herzfunktionssensoren, Elektrolytsensoren, Serumsensoren (z.B. Kreatinin), Neurosensoren (Vagus, Sympathikus), Adverse Event Detektoren, Worsening Heart Failure Sensoren, MRT-Sensoren, Aktivitätssensoren, Schlafapnoe-Sensoren, Ischemiesensoren, Sensoren für den methabolischen Bedarf und Infarktsensoren sowie Herzrhythmus-abhängigen CCM-Steuerungen allgemein beschrieben. In dem genannten Stand der Technik wird auch eine Abschaltung der CCM-Therapie bei erkanntem Vorhofflimmern bzw. Vorhofarrhythmien (siehe US2010/0069977 Fig. 20A und Absatz [0332]) beschrieben. Ebenso wird dort ganz allgemein auf die mögliche Kombination von CCM mit anderen Stimulations- und Elektrotherapieformen, wie CRT, ICD und Neurostimulation, eingegangen.

US 2010/0069977 A1, US 2010/0069980 A1, US 2010/0069984 A1, US 2010/0069985 A1 beschreiben eine Abschaltung der CCM-Therapie bei einem erkannten Vorhofflimmern bzw. Vorhofarrhythmien, allerdings wird nicht auf den Grund der CCM-Abschaltung eingegangen.

US 2010/0069977 A1, US 2010/0069980 A1, US 2010/0069984 A1, US 2010/0069985 A1 beschreiben auch die mögliche Kombination von CCM und CRT-Stimulation in einem Gerät.

US20090082825 beschreibt einen Herzstimulator gemäß dem ersten Teil des Anspruchs 1, zur Abgabe von high-output pacing Pulsen und Mitteln zur Messung des hämodynamischen Antwort. Die Messmittel umfassen unter anderem die Schallamplitude des Mitralklappe, systolischer Blutdruck oder Herzschlagvolumen.

CCM-Stimulationsimpulse werden immer in der absoluten Refraktärzeit eines jeweiligen Ventrikels abgegeben. So kann verhindert werden, dass diese Pulse eine Arrhythmie induzieren.

Das derzeit am Markt verfügbare CCM-System (Optimizer III von Impulse Dynamics) synchronisiert die CCM-Impulse über die Wahrnehmung der septalen Erregung mittels der lokalen CCM-Elektrode.

Der Erfinder hat folgende Nachteile bekannter CCM-Stimulatoren erkannt:
Wahrnehmungsfehler können bei der genannten Methode der CCM-Synchronisation dazu führen, dass die CCM-Impulse auch außerhalb der absoluten Refraktärzeit abgegeben werden könnten. Die kann eine proarrythmische Wirkung haben und eine Kammertachykardie oder Kammerflimmern induzieren.

Insbesondere der Einsatz der CCM-Stimulation zusammen mit zusätzlichen Stimulatoren, wie typischerweise einem biventriklulären Schrittmacher oder Defibrillator, erhöht dieses Risiko, da hier unter Umständen unterschwellige Stimuli abgegeben werden, die vom CCM-System aber wahrgenommen werden und eine CCM-Pulsabgabe triggern könnten.

Ein weiterer Nachteil der CCM-Therapie besteht in der Notwendigkeit, zwei zusätzliche Stimulationselektroden am Ventrikelseptum zu implantieren. Damit wird die Zahl der implantierten Elektroden bei gleichzeitigem Einsatz eines ICD oder CRT-D sehr groß.

Anders als bei anderen CRM-Stimulatoren ist bei den derzeitigen CCM-Systemen keine Stimulationserfolgskontrolle bekannt. Die Therapieparameter werden empirisch festgelegt und allenfalls nach dem klinischen Langzeitverlauf angepasst. Eine geräteinterne Therapieoptimierung ist daher bislang nicht vorhanden.

Der Erfindung liegt die Aufgabe zugrunde, aufbauend auf den von dem Erfinder erkannten Nachteilen des Standes der Technik, einen verbesserten Herzstimulator für die kardiale Kontraktilitätsmodulationstherapie zu schaffen.

Erfindungsgemäß wird diese Aufgabe durch einen Herzstimulator gemäß Anspruch 1 gelöst, der wenigstens eine Stimulationseinheit, die über wenigstens eine Elektrodenleitung mit wenigstens drei Stimulationselektrodenpolen verbunden oder zu verbinden ist und die ausgebildet ist, über wenigstens zwei Stimulationselektrodenpole unterschwellige Stimulationsimpulse für eine kardiale Kontraktionsmodulationstherapie abzugeben, aufweist sowie wenigstens eine Wahrnehmungseinheit zum Erfassen von elektrischen oder mechanischen Herzaktionen, die vorzugsweise mit den drei Stimulationselektrodenpolen verbunden oder zu verbinden ist. Die Wahrnehmungseinheit ist ausgebildet, für eine Herzaktion charakteristische Signale zu erfassen und weist eine Auswertungseinheit auf oder ist mit einer Auswertungseinheit verbunden, die ausgebildet ist, von der Wahrnehmungseinheit erfasste Signale auszuwerten und ein entsprechendes Auswertungsergebnissignal zu liefern. Außerdem weist der Herzstimulator eine Therapiesteuereinheit auf, die mit der Stimulationseinheit und der Auswertungseinheit verbunden ist und die ausgebildet ist, eine jeweilige kardiale Kontraktionsmodulationstherapie in Abhängigkeit eines jeweiligen Auswertungsergebnissignals der Auswerteeinheit zu steuern, und zwar vorzugsweise so, dass eine jeweilige kardiale Kontraktionsmodulationstherapie in Abhängigkeit eines Stimulationserfolgs oder Misserfolgs einer überschwelligen Stimulation aktiviert oder deaktiviert wird und/oder Therapieparameter einer jeweiligen kardialen Kontraktionsmodulationstherapie in Abhängigkeit des jeweiligen Auswertungsergebnissignals ausgewählt und/oder eingestellt werden. Dabei ist die Wahrnehmungseinheit so ausgebildet, dass sie die Dauer der Erregungsrückbildung an mindestens 2 Orten bestimmt.

Ein derartiger Herzstimulator bietet in einer Variante den Vorteil, dass er das proarrhythmische Risiko der CCM-Stimulation bei gleichzeitiger antibradykarder oder CRT-Stimulation signifikant senkt, indem eine CCM-Stimulation bei möglicherweise unsicherer Synchronisation infolge mangelnden Stimulationserfolgs einer überschwelligen Stimulation abgeschaltet wird.

Ein derartiger Herzstimulator bietet in einer anderen Variante außerdem den Vorteil, die CCM-Therapie automatisch und im Verlauf optimieren zu können.

Ein derartiger Herzstimulator bietet in Kombination beider Varianten den Vorteil, die CCM-Therapie automatisch und im Verlauf optimieren zu können und gleichzeitig eine potentiell proarrhythmische Wirkung der CCM-Stimulation in einem Kombinationstherapiegerät zu eliminieren.

Die vorgeschlagene Optimierung der CCM-Therapie durch die Therapiesteuereinheit in Reaktion auf von der Wahrnehmungseinheit erfasste Signale geht von dem CCM-Erklärungsmodell aus, dass die CCM-Stimulation in der absoluten Refraktärzeit eine Homogenisierung des Erregungszustandes der Myokardzellen (mindestens lokal) bewirkt und damit eine intra- und interventrikuläre Erregungssynchronisation gefördert wird. Zusätzlich ist anzunehmen, dass eine solche Homogenisierung auch über alternative Stimulationsvektoren hervorgerufen werden kann. Der für die automatische Therapieoptimierung notwendige Nachweis des CCM-Therapieerfolges kann durch Messung intra- und/oder interventrikulärer Erregungsausbreitungszeiten und oder Erregungsrückbildungszeiten nachgewiesen werden. Bei einem Therapieerfolg ist davon auszugehen, dass sich diese Zeiten, gemessen über verschiedene Vektoren, aneinander angleichen.

Für eine derartige Therapieoptimierung ist insbesondere ein implantierbares CCM-Therapiegerät mit mindestens 2 oder mehr CCM-Stimulationsstrompfaden vorgesehen, d.h. mindestens 3 oder mehr CCM-Stimulationselektrodenpolen, wobei mindestens 3 der CCM-Stimulationselektrodenpole und/oder weitere Wahrnehmungselektrodenpole mit einer Wahrnehmungseinheit verbunden sind und die Wahrnehmungseinheit ausgebildet ist, die Zeitpunkte einer charakteristischen intrinsischen oder stimulierten kardialen Erregung an den genannten Punkten zu bestimmen und eine Laufzeitmessung durchzuführen oder alternative Signalcharakteristika zu ermitteln. Eine mit der Wahrnehmungseinheit verbundene Therapiesteuereinheit ist ausgebildet, die CCM-Stimulationsvektoren und/oder Parameter in Abhängigkeit der Laufzeitmessung/Signalcharakteristika zu variern.

Die CCM-Stimulationsvektoren ergeben sich dabei vorzugsweise aus den bei einem CRT-System vorhandenen Elektrodenpolkombinationen.

Bevorzugte Ausführungsvarianten der Erfindung sind die folgenden:
Vorzugsweise ist die Wahrnehmungseinheit ausgebildet, für eine Herzaktion charakteristische Signale an dem jeweiligen Ort oder in unmittelbarer Nähe der drei Stimulationselektrodenpole zu erfassen. Hierbei ist die Wahrnehmungseinheit vorzugsweise ausgebildet, Zeitpunkte einer charakteristischen intrinsischen oder stimulierten kardialen Erregung an den drei Stimulationselektrodenpolen zu erfassen. Hierzu kann die Wahrnehmungseinheit mit den drei Stimulationselektrodenpolen und/oder diesen benachbarten Wahrnehmungselektrodenpolen verbunden oder zu verbinden sein und kann ausgebildet sein, über diese Elektrodenpole für eine Herzaktion charakteristische elektrische Potentiale (Aktionspotentiale) zu erfassen.

Wie schon angedeutet, weist die Wahrnehmungseinheit vorzugsweise eine Auswertungseinheit auf oder ist mit einer solchen verbunden, wobei die Auswertungseinheit ausgebildet ist, eine Laufzeit (Zeitdifferenz) zwischen wenigstens zwei von der Wahrnehmungseinheit erfassten Signalen zu bestimmen und ein entsprechendes Auswertungsergebnissignal zu liefern.

Alternativ oder zusätzlich ist es auch möglich, dass die Wahrnehmungseinheit eine Auswertungseinheit aufweist oder mit einer Auswertungseinheit verbunden ist, welche ausgebildet ist, Signalcharakteristika von wenigstens zwei von der Wahrnehmungseinheit erfassten Signalen zu bestimmen und ein entsprechendes Auswertungsergebnissignal zu liefern.

In allen Fällen kann die Therapiesteuereinheit ausgebildet sein, diejenigen zwei der Stimulationselektrodenpole für eine kardiale Kontraktionsmodulationstherapie auszuwählen, die jeweils zu ausgeglichenen intra- und/oder interventrikulären Erregungsausbreitungszeiten und/oder Erregungsrückbildungszeiten führen.

Erfindungsgemäß ist die Wahrnehmungseinheit ausgebildet die Dauer einer Erregungsrückbildung (z.B. Dauer der T-Welle) an mindestens zwei Orten zu bestimmen. Die Therapiesteuereinheit ist dann vorzugsweise ausgebildet, Therapieparameter einer kardialen Kontraktionsmodulationstherapie so auszuwählen und/oder einzustellen, dass sich ein Minimum der Erregungsrückbildungszeiten ergibt.

Alternativ oder zusätzlich kann die Therapiesteuereinheit auch ausgebildet sein, Therapieparameter einer kardialen Kontraktionsmodulationstherapie so auszuwählen und/oder einzustellen, dass sich eine Homogenisierung der Erregungsrückbildungszeiten ergibt.

Auch kann die Therapiesteuereinheit ausgebildet sein, Therapieparameter einer kardialen Kontraktionsmodulationstherapie so auszuwählen und/oder einzustellen, dass sich ein Maximum einer Anstiegsrate eines von der Wahrnehmungseinheit erfassten Aktionspotentials ergibt.

Gemäß einer weiteren Alternative oder einer zusätzlichen Möglichkeit ist die Therapiesteuereinheit ausgebildet, Therapieparameter einer kardialen Kontraktionsmodulationstherapie so auszuwählen und/oder einzustellen, dass sich eine Homogenisierung der Anstiegsraten von der Wahrnehmungseinheit erfasster Aktionspotentiale ergibt.

Auch kann es vorteilhaft sein, wenn die Therapiesteuereinheit ausgebildet ist, Therapieparameter einer kardialen Kontraktionsmodulationstherapie so auszuwählen und/oder einzustellen, dass sich ein Maximum der Aktionspotentialdauer eines von der Wahrnehmungseinheit erfassten Aktionspotentials ergibt.

Dementsprechend ist es bevorzugt, wenn die Wahrnehmungseinheit bzw. deren Auswertungseinheit ausgebildet ist, die Dauer der Erregungsrückbildung (z.B. Dauer der T-Welle) an mindestens zwei Punkten zu bestimmen. Das Therapieoptimum kann durch ein Minimum der Erregungsrückbildungszeiten definiert sein und/oder durch eine Homogenisierung der Erregungsrückbildungszeiten und/oder durch ein Maximum der Slew Rate des Aktionspotentials und/oder durch eine Homogenisierung der Slew Rate des Aktionspotentials und/oder durch ein Maximum der Aktionspotentialdauer und/oder durch eine Homogenisierung der Aktionspotentialdauer.

Gemäß einer besonders bevorzugten Ausführungsvariante weist der Herzstimulator wenigstens eine Stimulationseinheit zur Abgabe überschwelliger Stimulationsimpulse auf und die Auswerteinheit ist ausgebildet, von der Wahrnehmungseinheit nach Abgabe eines überschwelligen Stimulationsimpulses erfasste Signale hinsichtlich eines Stimulationserfolges auszuwerten und ein entsprechendes Auswertungsergebnissignal zu liefern. Die Therapiesteuereinheit ist hierbei ausgebildet, eine jeweilige kardiale Kontraktionsmodulationstherapie wenigstens für einen jeweiligen Herzzyklus zu deaktivieren, falls das Auswertungsergebnissignal keinen Stimulationserfolg anzeigt und/oder eine jeweilige kardiale Kontraktionsmodulationstherapie für wenigstens einen jeweiligen Herzzyklus nur dann zulässt, wenn das Auswertungsergebnissignal eine wirksame Stimulation an wenigstens einem oder allen genutzten ventrikulären Stimulationsorten anzeigt.

Wie bereits erwähnt, bietet ein derartiger Herzstimulator den Vorteil, dass er das proarrhythmische Risiko der CCM-Stimulation bei gleichzeitiger antibradykarder oder CRT-Stimulation signifikant senkt, indem eine CCM-Stimulation bei möglicherweise unsicherer Synchronisation infolge mangelnden Stimulationserfolgs einer überschwelligen Stimulation abgeschaltet wird.

Vorzugsweise ist der Herzstimulator somit ein implantierbares Kombinationstherapiegerät zur CCM-Therapie und zur gleichzeitigen antibradykarden und/oder CRT-Stimulation, das eine CCM-Stimulations- und Synchronisationseinheit und eine CRT/Bardykardie-Stimulationseinheit aufweist, und der zusätzlich in Form der Wahrnehmungseinheit und der entsprechend ausgebildeten Auswerteinheit eine Stimulationserfolgskontrolleinheit mindestens für die ventrikuläre Stimulation aufweist. Die Therapiesteuereinheit ist dann so konfiguriert, dass sie eine CCM-Stimulation im laufenden Herzzyklus nur dann zulässt, wenn zuvor die Stimulationserfolgskontrolleinheit eine effektive Stimulation aller genutzter ventrikulärer Stimulationsorte angezeigt hat. Andernfalls wird die CCM-Stimulation ausgesetzt.

Vorzugsweise erfolgt Stimulationserfolgskontrolle durch die Wahrnehmungseinheit und die Auswerteinheit mittels Auswertung evozierten Potentials an der jeweiligen Elektrode. Alternativ oder zusätzlich kann die Stimulationserfolgskontrolle mittels Auswertung eines CLS-Signals erfolgen. Eine weitere Alternative ist eine Stimulationserfolgskontrolle mittels Auswertung eines HDS-Signals, d.h. durch Nachweis einer tatsächlichen Kontraktion. Vorzugsweise weist der Herzstimulator außerdem einen Speicher auf und ist so ausgebildet, dass eine Inhibierung der CCM-Stimulation in einer Statistik zusammen mit dem Grund für die Inhibierung gespeichert wird.

Insgesamt ergibt sich ein Herzstimulator für eine verbesserte CCM-Therapie, der mittels automatisch umschaltbarer CCM-Strompfade, einer CCM-Effektivitätskontrolle und einer CCM-Sicherheitsstimulationseinrichtung die CCM-Therapie automatisch optimiert.

Die Erfindung soll nun anhand eines Ausführungsbeispiels mit Bezug auf die Figuren näher erläuter werden. Von den Figuren zeigt
- Fig. 1: einen CRT-CCM-Stimulator;
- Fig. 2: ein Blockschaltbild für eine automatische Therapieoptimierung;
- Fig. 3: ein Beispiel eines Aktionspotentials bei ineffektiver CCM-Stimulation;
- Fig. 4: ein Beispiel eines Aktionspotentials bei effektiver CCM-Stimulation; und
- Fig. 5: ein Blockschaltbild für eine CCM-Inhibierung bei mangelndem Stimulationserfolg einer CRM-Stimulation.

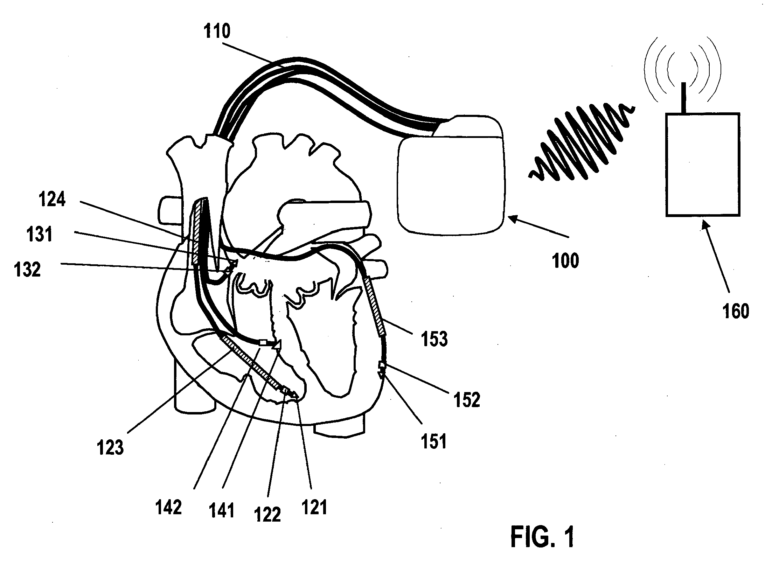

In der Figur 1 ist als Implementierungsbeispiel eines Herzstimulators ein Dreikammer-ICD-System mit integrierter CRT-CCM-Funktion dargestellt. Ein Generator 100 ist mit mehreren implantierbaren Elektrodenleitungen 110 verbunden. Zur rechtsventrikulären Wahrnehmung und Stimulation umfasst eine rechtsventrikuläre Elektrodenleitung eine rechtsventrikuläre Tipelektrode 121 und eine rechtsventrikuläre Ringelektrode 122 am distalen Ende der rechtsventrikulären Elektrodenleitung. Über die rechtsventrikuläre Tipelektrode 121 werden im Falle einer kardialen Resynchronisationstherapie (CRT) rechtsventrikuläre Stimulationsimpulse für die biventrikuläre CRT-Stimulation abgegeben. Zur Abgabe von Defibrillationsschock sind an dieser rechtsventrikulären Elektrodenleitung eine distale Schockwendel 123 und optional auch eine proximale Schockwendel 124 angebracht. Die Gegenelektrode für die Abgabe von Defibrillationsschocks bildet hier das Generatorgehäuse 100.

Eine rechtsatriale Elektrodenleitung weist an ihrem distalen Ende einen bipolaren Wahrnehmungs- und Stimulationspol mit einer rechtsatrialen Tipelektrode 131 und einer rechtsatrialen Ringelektrode 132 auf und dient der Wahrnehmung des atrialen Rhythmus und bei Bedarf der atrialen Stimulation.

Außerdem umfasst das System auch eine linksventrikuläre bzw. CS(Coronar Sinus)-Elektrode zur Angabe von linksventrikulären Stimulationsimpulsen zur CRT über eine linksventrikuläre Tipelektrode 151 und eine linksventrikuläre Ringelektrode 152. Zur effektiveren Defibrillation/Kardioversion ist optional eine linksventrikuläre Schockwendel 153 vorgesehen.

Zur CCM-Stimulation sind ein oder mehrere rechtsventrikulär-septale Elektrodenleitungen vorgesehen, die über eine septale Tipelektrode 141 und eine septale Ringelektrode 142 die CCM-Pulse jeweils an das ventrikuläre Septum abgeben. Es können jedoch auch mehr als zwei septale CCM-Stimulationspole oder aber die weiteren zur CRT-D-Stimulation genutzten Elektrodenpole (121, 122, 123, 151, 152, 153) für die CCM-Vektorumschaltung genutzt werden.

Eine Wahrnehmungs- und Auswerteeinheit (250 ... 280; 580) ist mit mindestens zwei und vorzugsweise allen der Stimulationselektrodenpole (121, 122, 141, 142, 151, 152) verbunden, um die lokalen Erregungsausbreitungsgeschwindigkeiten und Erregungsrückbildungsparameter zu erfassen.

Für Kommunikation mit externen Programmier- und Steuer- und Datenübertragungsgeräten 160 ist eine drahtlose bidirektionale Telemetrieeinheit vorgesehen.

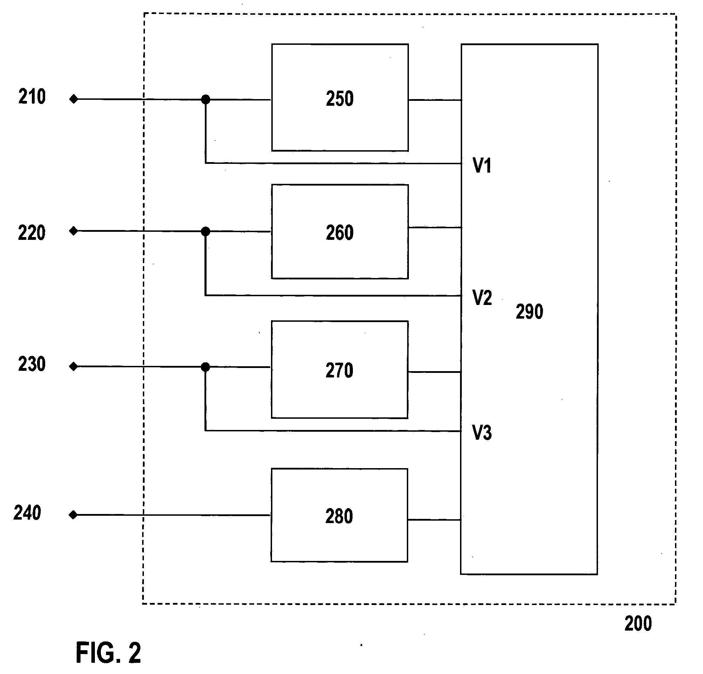

In Figur 2 ist ein Blockschaltbild mit denjenigen Komponenten des Herzstimulators dargestellt, die einer automatischen Optimierung einer CCM-Therapie dienen. Ein CCM-Generator 200 ist hier mit insgesamt vier Elektrodenpolen (210...240) verbunden. Für die CCM-Stimulationsimpulsabgabe können drei Elektrodenpole (210...230) wahlweise genutzt werden, so dass sich umschaltbare Stimulations-Vektoren ergeben. Alle vier Elektrodenpole sind mit jeweils einer baugleichen Wahrnehmungseinheit verbunden (250...280). Diese Wahrnehmungseinheiten schließen eine nicht explizit dargestellte Auswerteinheit ein und erfassen vorzugsweise einen Parameter der Erregungsrückbildung aus dem intrakardialen Elektrogramm am jeweiligen Elektrodenpol und leiten diese Information in Form eines jeweiligen Auswertungsergebnissignals an eine CCM-Therapiesteuereinheit 290 weiter. Die CCM-Therapiesteuereinheit 290 wertet die Informationen über die Erregungsrückbildung aus und optimiert mittels eines gängigen Suchverfahrens die für die CCM-Therapie optimale Impulsparametrisierung, insbesondere bestimmt sie den wirkungsvollsten und energetisch günstigsten Stimulationsvektor (V1...V3).

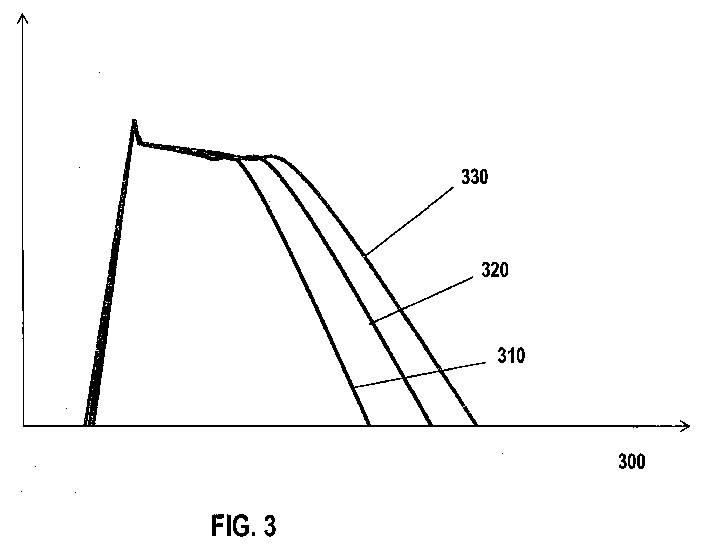

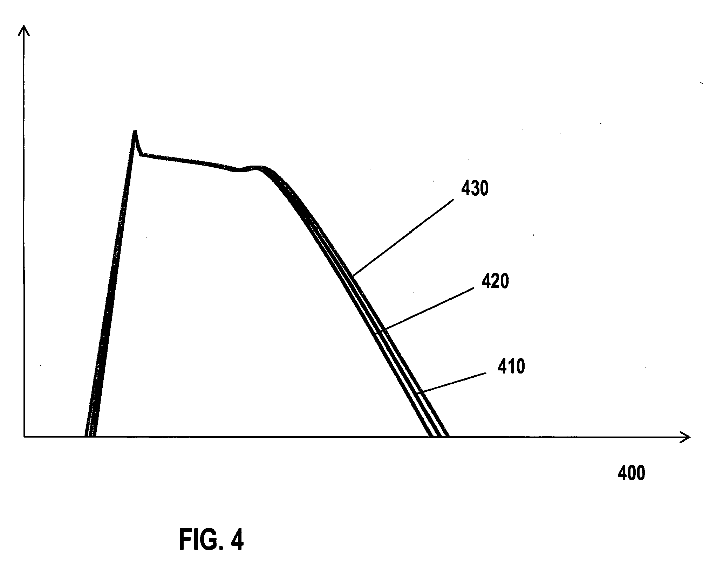

Figuren 3 und 4 zeigen am Beispiel von Aktionspotentialverläufen eine angenommene Regelgröße für das Implementierungsbeispiel.

In dem in Figur 3 dargestellten Beispiel ist die Erregungsrückbildung relativ breit gestreut (310..330), so dass hier von einen weniger effektiven CCM-Therapie ausgegangen werden kann.

Anders in dem in Figur 4 dargestellten Beispiel: Hier sind die Erregungsrückbildungen im Vergleich wesentlich homogener (410...430), so dass damit ein positiver Effekt der CCM-Therapie demonstriert wird.

Alternativ kann die CCM-Therapiesteuereinheit als Regelgröße auch die Signallaufzeiten verschiedener Erregungsabschnitte an den einzelnen Elektrodenpolen oder zwischen verschiedenen Vektoren vergleichen. Ziel der Therapieoptimierung ist immer eine Homogenisierung der Signallaufzeiten.

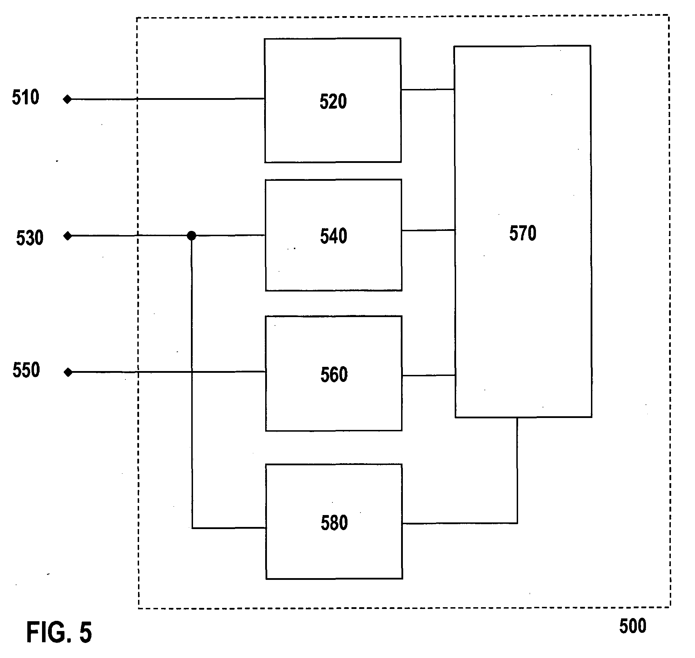

In Figur 5 sind separat in einem Blockschaltbild diejenigen Komponenten eines kombinierten CRT-CCM-Stimulators dargestellt, die der CCM-Therapie-Inhibierung im Falle einer erfolglosen CRT-Stimulation dienen. Der in Figur 5 abgebildete CRT-CCM-Stimulator umfasst mindestens folgende Komponenten: einen Generator 500; eine rechtsatriale Elektrodenleitung 510, verbunden mit einer atrialen Wahrnehmungs- und Stimulationseinheit zur Klassifikation des atrialen Rhythmus und bedarfsgesteuerten atrialen Stimulation; rechtsventrikuläre und linksventrikuläre Elektrodenanschlüsse 530, verbunden mit einer biventrikulären Wahrnehmungs- und Stimulationseinheit zur Wahrnehmung und Klassifikation des ventrikulären Rhythmus und der biventrikulären CRT-Stimulation sowie einer oder mehrerer CCM-Elektroden, verbunden mit einer CCM-Stimulationseinheit 560, wobei die atriale Wahrnehmungs- und Stimulationseinheit 520, die biventrikuläre Wahrnehmungs- und Stimulationseinheit 540 und die CCM-Stimulationseinheit 560 zur Steuerung und Synchronisation der CRT- und CCM-Therapie mit einer Therapiesteuereinheit 570 verbunden sind.

Zusätzlich umfasst der kombinierte CRT-CCM-Stimulator eine Stimulationserfolgskontrolleinheit 580, ebenfalls verbunden mit den ventrikulären Elektrodenanschlüssen 530. Diese Einheit bewertet den Stimulationserfolg der ventrikulären Stimulation für jeden Zyklus und jede ventrikuläre Elektrode und gibt diese Bewertung an die Therapiesteuereinheit 570 weiter.

Die Therapiesteuereinheit inhibiert die CCM-Stimulation immer dann, wenn an wenigstens einer der ventrikulären Elektroden der Stimulationserfolg im aktuellen Herzzyklus ausgeblieben ist.

Das Blockschaltbild aus Figur 5 ist mit dem Blockschaltbild aus Figur 2 zu kombinieren.

Ein hier beschriebener Herzstimulator hat den Vorteil, dass die CCM-Therapie automatisch vom Implantat optimiert werden kann und ggf. die Anzahl den benötigten Elektrodenleitungen in einem Kombinationstherapiegerät reduziert werden kann.

Ein solcher Herzstimulator bietet außerdem den Vorteil, dass damit ohne proarrhythmisches Risiko die CCM-Stimulation in einem Kombinationstherapiegerät gleichzeitig mit einer CRT-Stimulation oder antibradykarden Stimulation eingesetzt werden kann.

## Patentansprüche

1. Herzstimulator (100) mit
wenigstens einer Stimulationseinheit (200), die über wenigstens eine Elektrodenleitung (110) mit wenigstens drei Stimulationselektrodenpolen (121, 122, 141, 142, 151, 152, 210, 220, 230) verbunden oder zu verbinden ist und die ausgebildet ist, über wenigstens zwei Stimulationselektrodenpole unterschwellige Stimulationsimpulse für eine kardiale Kontraktionsmodulationstherapie abzugeben,
sowie mit wenigstens einer Wahrnehmungseinheit (250, 260, 270, 280) zum Erfassen von elektrischen oder mechanischen Herzaktionen,
wobei,
die Wahrnehmungseinheit (250, 260, 270, 280) ausgebildet ist, für eine Herzaktion charakteristische Signale zu erfassen und eine Auswertungseinheit aufweist oder mit einer Auswertungseinheit verbunden ist, die ausgebildet ist, von der Wahrnehmungseinheit (250, 260, 270, 280) erfasste Signale auszuwerten und ein entsprechendes Auswertungsergebnissignal zu liefern, und
der Herzstimulator (100) eine Therapiesteuereinheit (290, 570) aufweist, die mit der Stimulationseinheit (200) und der Auswertungseinheit verbunden ist und die ausgebildet ist, eine jeweilige kardiale Kontraktionsmodulationstherapie in Abhängigkeit eines jeweiligen Auswertungsergebnissignals der Auswerteeinheit derart zu steuern, dass eine jeweilige kardiale Kontraktionsmodulationstherapie in Abhängigkeit eines Stimulationserfolgs oder Misserfolgs einer überschwelligen Stimulation aktiviert oder deaktiviert wird und/oder Therapieparameter einer jeweiligen kardialen Kontraktionsmodulationstherapie in Abhängigkeit des jeweiligen Auswertungsergebnissignals ausgewählt und/oder eingestellt werden, **dadurch gekennzeichnet, dass** die Wahrnehmungseinheit (210, 220, 230) ausgebildet ist, die Dauer einer Erregungsrückbildung an mindestens zwei Orten zu bestimmen.

2. Herzstimulator (100) nach Anspruch 1, **dadurch gekennzeichnet, dass** die Wahrnehmungseinheit (250, 260, 270, 280) ausgebildet ist, für eine Herzaktion charakteristische Signale an dem jeweiligen Ort oder in unmittelbarer Nähe der drei Stimulationselektrodenpole (210, 220, 230) zu erfassen.

3. Herzstimulator nach Anspruch 2, **dadurch gekennzeichnet, dass** die Wahrnehmungseinheit (250, 260, 270, 280) ausgebildet ist, Zeitpunkte einer charakteristischen intrinsischen oder stimulierten kardialen Erregung an den drei Stimulationselektrodenpolen (210, 220, 230) zu erfassen.

4. Herzstimulator (100) nach Anspruch 3, **dadurch gekennzeichnet, dass** die Wahrnehmungseinheit (250, 260, 270, 280) mit den drei Stimulationselektrodenpolen (210, 220, 230) und/oder diesen benachbarten Wahrnehmungselektrodenpolen verbunden oder zu verbinden ist und ausgebildet ist, über diese Elektrodenpole für eine Herzaktion charakteristische elektrische Potentiale zu erfassen.

5. Herzstimulator (100) nach einem der Ansprüche 1 bis 4, **dadurch gekennzeichnet, dass** die Wahrnehmungseinheit (250, 260, 270, 280) eine Auswertungseinheit aufweist oder mit einer Auswertungseinheit verbunden ist, welche ausgebildet ist, eine Zeitdifferenz zwischen wenigstens zwei von der Wahrnehmungseinheit (250, 260, 270, 280) erfassten Signalen zu bestimmen und ein entsprechendes Auswertungsergebnissignal zu liefern.

6. Herzstimulator (100) nach einem der Ansprüche 1 bis 4, **dadurch gekennzeichnet, dass** die Wahrnehmungseinheit (250, 260, 270, 280) eine Auswertungseinheit aufweist oder mit einer Auswertungseinheit verbunden ist, welche ausgebildet ist, Signalcharakteristika von wenigstens zwei von der Wahrnehmungseinheit (250, 260, 270, 280) erfassten Signalen zu bestimmen und ein entsprechendes Auswertungsergebnissignal zu liefern.

7. Herzstimulator (100) nach wenigstens einem der Ansprüche 5 oder 6, **dadurch gekennzeichnet, dass** die Therapiesteuereinheit (290, 570) ausgebildet ist, diejenigen zwei der Stimulationselektrodenpole (210, 220, 230) für eine kardiale Kontraktionsmodulationstherapie auszuwählen, die jeweils zu ausgeglichenen intra-und/oder interventrikulären Erregungsausbreitungszeiten und/oder Erregungsrückbildungszeiten führen.

8. Herzstimulator (100) nach Anspruch 1, **dadurch gekennzeichnet, dass** die Therapiesteuereinheit (290, 570) ausgebildet ist, Therapieparameter einer kardialen Kontraktionsmodulationstherapie so auszuwählen und/oder einzustellen, dass sich ein Minimum der Erregungsrückbildungszeiten ergibt.

9. Herzstimulator (100) nach Anspruch 1 oder 8, **dadurch gekennzeichnet, dass** die Therapiesteuereinheit (290, 570) ausgebildet ist, Therapieparameter einer kardialen Kontraktionsmodulationstherapie so auszuwählen und/oder einzustellen, dass sich eine Homogenisierung der Erregungsrückbildungszeiten ergibt.

10. Herzstimulator (100) nach wenigstens einem der Ansprüche 1, 8 und 9, **dadurch gekennzeichnet, dass** die Therapiesteuereinheit (290, 570) ausgebildet ist, Therapieparameter einer kardialen Kontraktionsmodulationstherapie so auszuwählen und/oder einzustellen, dass sich ein Maximum einer Anstiegsrate eines von der Wahrnehmungseinheit (210, 220, 230) erfassten Aktionspotentials ergibt.

11. Herzstimulator (100) nach wenigstens einem der Ansprüche 1 und 8 bis 10, **dadurch gekennzeichnet, dass** die Therapiesteuereinheit (290, 570) ausgebildet ist, Therapieparameter einer kardialen Kontraktionsmodulationstherapie so auszuwählen und/oder einzustellen, dass sich eine Homogenisierung der Anstiegsraten von der Wahrnehmungseinheit (210, 220, 230) erfasster Aktionspotentiale ergibt.

12. Herzstimulator (100) nach wenigstens einem der Anspruch1 und 8 bis 11, **dadurch gekennzeichnet, dass** die Therapiesteuereinheit (290, 570) ausgebildet ist Therapieparameter einer kardialen Kontraktionsmodulationstherapie so auszuwählen und/oder einzustellen, dass sich ein Maximum der Aktionspotentialdauer eines von der Wahrnehmungseinheit (210, 220, 230) erfassten Aktionspotentials ergibt.

13. Herzstimulator (100) nach wenigstens einem der Ansprüche 1 und 8 bis 12, **dadurch gekennzeichnet, dass** die Therapiesteuereinheit (290, 570) ausgebildet ist, Therapieparameter einer kardialen Kontraktionsmodulationstherapie so auszuwählen und/oder einzustellen, dass sich eine Homogenisierung der Aktionspotentialdauer von der Wahrnehmungseinheit (210, 220, 230) erfasster Aktionspotentiale ergibt.

14. Herzstimulator (100) nach einem der Ansprüche 1 bis 13, **dadurch gekennzeichnet, dass** der Herzstimulator (100) wenigstens eine Stimulationseinheit (200) zur Abgabe überschwelliger Stimulationsimpulse aufweist und die Auswerteinheit ausgebildet ist, von der Wahrnehmungseinheit (210, 220, 230) nach Abgabe eines überschwelligen Stimulationsimpulses erfasste Signale hinsichtlich eines Stimulationserfolges auszuwerten und ein entsprechendes Auswertungsergebnissignal zu liefern, und
die Therapiesteuereinheit (290, 570) ausgebildet ist, eine jeweilige kardiale Kontraktionsmodulationstherapie wenigstens für einen jeweiligen Herzzyklus zu deaktivieren, falls das Auswertungsergebnissignal keinen Stimulationserfolg anzeigt und/ oder
eine jeweilige kardiale Kontraktionsmodulationstherapie für wenigstens einen jeweiligen Herzzyklus nur dann zulässt, wenn das Auswertungsergebnissignal eine wirksame Stimulation an wenigstens einem oder allen genutzten ventrikulären Stimulationsorten anzeigt.

## Claims

1. A cardiac stimulator (100) comprising
at least one stimulation unit (200), which is connected or is to be connected to at least three stimulation electrode poles (121, 122, 141, 142, 151, 152, 210, 220, 230) via at least one electrode lead (110) and which is configured to deliver sub-threshold stimulation pulses for a cardiac contractility modulation therapy via at least two stimulation electrode poles,
and comprising at least one sensing unit (250, 260, 270, 280) for detecting electrical or mechanical cardiac actions,
wherein
the sensing unit (250, 260, 270, 280) is configured to detect signals characteristic of a cardiac action and has an evaluation unit or is connected to an evaluation unit configured to evaluate signals detected by the sensing unit (250, 260, 270, 280) and to supply a corresponding evaluation result signal, and
the cardiac stimulator (100) has a therapy control unit (290, 570) connected to the stimulation unit (200) and the evaluation unit and is configured to control a respective cardiac contractility modulation therapy depending on a respective evaluation result signal of the evaluation unit, in such a way that a respective cardiac contractility modulation therapy is activated or deactivated depending on a stimulation success or failure of a supra-threshold stimulation, and/or therapy parameters of a respective cardiac contractility modulation therapy are selected and/or adjusted depending on the respective evaluation result signal, **characterised in that** the sensing unit (210, 220, 230) is configured to determine the duration of a repolarisation at least at two sites.

2. The cardiac stimulator (100) according to Claim 1, **characterised in that** the sensing unit (250, 260, 270, 280) is configured to detect signals characteristic of cardiac action at the respective site or in the direct vicinity of the three stimulation electrode poles (210, 220, 230).

3. The cardiac stimulator according to Claim 2, **characterised in that** the sensing unit (250, 260, 270, 280) is configured to detect times of a characteristic intrinsic or stimulated cardiac excitation at the three stimulation electrode poles (210, 220, 230).

4. The cardiac stimulator (100) according to Claim 3, **characterised in that** the sensing unit (250, 260, 270, 280) is connected or is to be connected to the three stimulation electrode poles (210, 220, 230) and/or sensing electrode poles adjacent thereto and is configured to detect electrical potentials characteristic of cardiac action via these electrode poles.

5. The cardiac stimulator (100) according to one of Claims 1 to 4, **characterised in that** the sensing unit (250, 260, 270, 280) has an evaluation unit or is connected to an evaluation unit configured to determine a time lag between at least two signals detected by the sensing unit (250, 260, 270, 280) and to supply a corresponding evaluation result signal.

6. The cardiac stimulator (100) according to one of Claims 1 to 4, **characterised in that** the sensing unit (250, 260, 270, 280) has an evaluation unit or is connected to an evaluation unit configured to determine signal characteristics of at least two signals detected by the sensing unit (250, 260, 270, 280) and to supply a corresponding evaluation result signal.

7. The cardiac stimulator (100) according to at least one of Claims 5 or 6, **characterised in that** the therapy control unit (290, 570) is configured to select those two of the stimulation electrode poles (210, 220, 230) for a cardiac contractility modulation therapy that result in balanced intraventricular and/or interventricular depolarisation times and/or repolarisation times.

8. The cardiac stimulator (100) according to Claim 1, **characterised in that** the therapy control unit (290, 570) is configured to select and/or adjust therapy parameters of a cardiac contractility modulation therapy such that a minimum of repolarization times is obtained.

9. The cardiac stimulator (100) according to Claim 1 or 8, **characterised in that** the therapy control unit (290, 570) is configured to select and/or adjust therapy parameters of a cardiac contractility modulation therapy such that a homogenisation of the repolarisation times is obtained.

10. The cardiac stimulator (100) according to at least one of Claims 1, 8 and 9, **characterised in that** the therapy control unit (290, 570) is configured to select and/or adjust therapy parameters of a cardiac contractility modulation therapy such that a maximum of a rate of increase of an action potential detected by the sensing unit (210, 220, 230) is obtained.

11. The cardiac stimulator (100) according to at least one of Claims 1 and 8 to 10, **characterised in that** the therapy control unit (290, 570) is configured to select and/or adjust therapy parameters of a cardiac contractility modulation therapy such that a homogenisation of the rates of increase of action potentials detected by the at least one sensing unit (210, 220, 230) is obtained.

12. The cardiac stimulator (100) according to at least one of Claims 1 and 8 to 11, **characterised in that** the therapy control unit (290, 570) is configured to select and/or adjust therapy parameters of a cardiac contractility modulation therapy such that a maximum of an action potential duration of an action potential detected by the sensing unit (210, 220, 230) is obtained.

13. The cardiac stimulator (100) according to at least one of Claims 1 and 8 to 12, **characterised in that** the therapy control unit (290, 570) is configured to select and/or adjust therapy parameters of a cardiac contractility modulation therapy such that a homogenisation of the action potential duration of action potentials detected by the sensing unit (210, 220, 230) is obtained.

14. The cardiac stimulator (100) according to one of Claims 1 to 13, **characterised in that** the cardiac stimulator (100) has at least one stimulation unit (200) for delivering supra-threshold stimulation pulses and the evaluation unit is configured to evaluate signals detected by the sensing unit (210, 220, 230) after delivery of a supra-threshold stimulation pulse with respect to a success of the stimulation and to supply a corresponding evaluation result signal, and
the therapy control unit (290, 570) is configured to deactivate a respective cardiac contractility modulation therapy at least for one respective cardiac cycle if the evaluation result signal does not show any stimulation success, and/or
to allow a respective cardiac contractility modulation therapy for at least one respective cardiac cycle only if the evaluation result signal shows an effective stimulation at least at one ventricular stimulation site, or all used sites.

## Revendications

1. Stimulateur cardiaque (100) avec
au moins une unité de stimulation (200), qui est reliée, est à relier, avec au moins trois pôles d'électrodes de stimulation (121, 122, 141, 142, 151, 152, 210, 220, 230) par l'intermédiaire d'au moins une ligne d'électrode (110) et qui est conçue pour délivrer, par l'intermédiaire d'au moins deux pôles d'électrodes de stimulation, des impulsions de stimulation en dessous d'un seuil pour une thérapie de modulation des contractions cardiaques, ainsi qu'avec au moins une unité de détection (250, 260, 270, 280) pour l'évaluation d'actions électriques ou mécaniques sur le coeur,
dans lequel
l'unité de détection (250, 260, 270, 280) est conçue pour détecter des signaux caractéristiques d'une activité sur le coeur et présente une unité d'évaluation, ou est reliée avec une unité d'évaluation, qui est conçue pour traiter des signaux détectés par l'unité de détection (250, 260, 270, 280) et délivrer un signal de résultat d'évaluation correspondant, et
le stimulateur cardiaque (100) présente une unité de commande thérapeutique (290, 570) qui est reliée avec l'unité de stimulation (200) et avec l'unité d'évaluation et qui est conçue pour commander une thérapie spécifique de modulation des contractions cardiaques en fonction d'un signal de résultat d'évaluation spécifique de l'unité d'évaluation de telle manière qu'une thérapie de modulation des contractions cardiaques correspondante est activée ou désactivée en fonction du succès de la stimulation ou de l'échec d'une stimulation au dessus du seuil, et/ou choisit, et/ou règle, les paramètres de thérapie spécifiques d'une thérapie de modulation des contractions cardiaques spécifique en fonction du signal de résultat d'évaluation spécifique, **caractérisé en ce que** l'unité de détection (210, 220, 230) est conçue pour estimer la durée d'une repolarisation à au moins deux endroits.

2. Stimulateur cardiaque (100) selon la revendication 1, **caractérisé en ce que** l'unité de détection (250, 260, 270, 280) est conçue pour détecter des signaux caractéristiques pour une activité sur le coeur à l'endroit ou à proximité immédiate des trois pôles d'électrodes de stimulation (210, 220, 230).

3. Stimulateur cardiaque selon la revendication 2, **caractérisé en ce que** l'unité de détection (250, 260, 270, 280) est conçue pour évaluer les instants pour une stimulation cardiaque caractéristique intrinsèque ou stimulée sur les trois pôles d'électrodes de stimulation (210, 220, 230).

4. Stimulateur cardiaque (100) selon la revendication 3, **caractérisé en ce que** l'unité de détection (250, 260, 270, 280) est reliée avec les trois pôles d'électrodes de stimulation (210, 220, 230), et/ou est reliée, ou est à relier, aux pôles d'électrodes de détection voisins de ceux-ci et est conçue pour déterminer les potentiels électriques caractéristiques pour une action sur le coeur par l'intermédiaire de ces pôles d'électrodes.

5. Stimulateur cardiaque (100) selon l'une des revendications 1 à 4, **caractérisé en ce que** l'unité de détection (250, 260, 270, 280) présente une unité d'évaluation, ou est reliée avec une unité d'évaluation, laquelle est conçue pour déterminer une différence de temps entre au moins deux des signaux déterminés par l'unité de détection (250, 260, 270, 280) et délivrer un signal de résultat d'évaluation approprié.

6. Stimulateur cardiaque (100) selon l'une des revendications 1 à 4, **caractérisé en ce que** l'unité de détection (250, 260, 270, 280) présente une unité d'évaluation, ou est reliée avec une unité d'évaluation, laquelle est conçue pour déterminer les caractéristiques de signal d'au moins deux signaux saisis par l'unité de détection (205, 260, 270, 280) et délivrer un signal de résultat d'évaluation correspondant.

7. Stimulateur cardiaque (100) selon au moins l'une des revendications 5 ou 6, **caractérisé en ce que** l'unité de commande thérapeutique (290, 570) est conçue pour sélectionner les deux pôles d'électrodes de stimulation (210, 220, 230) pour une thérapie de modulation des contractions cardiaques qui conduisent respectivement à des temps de développement de stimulation intra- et/ou interventriculaires et/ou à des temps de repolarisation équilibrés.

8. Stimulateur cardiaque (100) selon la revendication 1, **caractérisé en ce que** l'unité de commande thérapeutique (290, 570) est conçue pour sélectionner, et/ou pour régler, les paramètres de thérapie d'une thérapie de modulation des contractions cardiaques de telle manière qu'il y ait un minimum de temps de repolarisation.

9. Stimulateur cardiaque (100) selon les revendications 1 ou 8, **caractérisé en ce que** l'unité de commande thérapeutique (290, 570) est conçue pour sélectionner, et/ou pour régler, les paramètres de thérapie d'une thérapie de modulation des contractions cardiaques de telle manière qu'il y ait une homogénéisation des temps de repolarisation.

10. Stimulateur cardiaque (100) selon au moins l'une des revendications 1, 8 et 9, **caractérisé en ce que** l'unité de commande thérapeutique (290, 570) est conçue pour sélectionner, et/ou pour régler, les paramètres de thérapie d'une thérapie de modulation des contractions cardiaques de telle manière qu'il y ait un maximum d'un taux de croissance d'un potentiel d'action relevé par l'unité de détection (210, 220, 230).

11. Stimulateur cardiaque (100) selon au moins l'une des revendications 1, 8 à 10, **caractérisé en ce que** l'unité de commande thérapeutique (290, 570) est conçue pour sélectionner, et/ou pour régler, les paramètres de thérapie d'une thérapie de modulation des contractions cardiaques de telle manière qu'il y ait une homogénéisation des taux de croissance des potentiels d'action relevés par l'unité de détection (210, 220, 230).

12. Stimulateur cardiaque (100) selon au moins l'une des revendications 1, 8 à 11, **caractérisé en ce que** l'unité de commande thérapeutique (290, 570) est conçue pour sélectionner, et/ou pour régler, les paramètres de thérapie d'une thérapie de modulation des contractions cardiaques de telle manière qu'il y ait un maximum de la durée du potentiel d'action d'un potentiel d'action relevé par l'unité de détection (210, 220, 230).

13. Stimulateur cardiaque (100) selon au moins l'une des revendications 1, 8 à 12, **caractérisé en ce que** l'unité de commande thérapeutique (290, 570) est conçue pour sélectionner, et/ou pour régler, les paramètres de thérapie d'une thérapie de modulation des contractions cardiaques de telle manière qu'il y ait une homogénéisation de la durée de potentiel d'action des potentiels d'action relevés par l'unité de détection (210, 220, 230).

14. Stimulateur cardiaque (100) selon l'une des revendications 1 à 13, **caractérisé en ce que** le stimulateur cardiaque (100) présente au moins une unité de stimulation (200) pour la délivrance d'impulsions de stimulation dépassant un seuil et que l'unité d'évaluation est conçue pour évaluer des signaux relevés après la délivrance d'une impulsion de stimulation au-delà d'un seuil en ce qui concerne un succès de la stimulation et pour délivrer un signal de résultat d'évaluation correspondant, et
l'unité de commande thérapeutique (290, 570) est conçue pour désactiver une thérapie de modulation de contractions cardiaques spécifique pour un cycle cardiaque spécifique dans le cas où le signal de résultat d'évaluation indique un échec de la stimulation,
et/ou
pour ne permettre une thérapie de modulation des contractions cardiaques spécifique pour au moins un cycle cardiaque spécifique uniquement si le signal de résultat d'évaluation indique une stimulation efficace sur au moins un ou tous les lieux de stimulation ventriculaire utilisés.
